# EUROPÄISCHE PATENTSCHRIFT

(11) **EP 1 133 495 B1**
(45) Veröffentlichungstag und Bekanntmachung des Hinweises auf die Patenterteilung: **09.10.2002**
(21) Anmeldenummer: 99955995.8
(22) Anmeldetag: 15.11.1999
(51) Int. Cl.: C07D 471/04, A61K 31/4709, A61P 31/04

(54) **SEMI-HYDROCHLORID VON 8-CYAN-1- CYCLOPROPYL -7-(1S,6S-2, 8-DIAZABICYCLO 4.3.0]NONAN -8-YL)-6- FLUOR-1, 4-DIHYDRO-4- OXO-3- CHINOLINCARBONSÄURE**
SEMI-HYDROCHLORIDE OF 8-CYAN-1- CYCLOPROPYL -7-(1S,6S-2 ,8-DIAZABICYCLO 4.3.0]NONAN -8-YL)-6- FLUORO-1, 4-DIHYDRO -4-OXO-3- QUINOLINE CARBOXYLIC ACID
SEMI-HYDROCHLORURE D'ACIDE 8-CYAN-1-CYCLOPROPYL-7-(1S,6S-2,8-DIAZABICYCLO 4.3.0]NONAN-8-YL)-6-FLUORO-1,4-DIHYDRO-4-OXO-3-CHINOLINCARBOXYLIQUE

(30) Priorität: 25.11.1998 DE 19854357
(43) Veröffentlichungstag der Anmeldung: 19.09.2001
(73) Patentinhaber: Bayer Aktiengesellschaft, 51368 Leverkusen (DE)
(72) Erfinder: HIMMLER, Thomas, D-51519 Odenthal (DE); RAST, Hubert, D-51381 Leverkusen (DE)
(86) Internationale Anmeldenummer: EP9908778
(87) Internationale Veröffentlichungsnummer: WO00031077

(56) Entgegenhaltungen:
- WO-A-97/31001

## Beschreibung

Die vorliegende Erfindung betrifft ein Semi-Hydrochlorid von 8-Cyan-1-cyclopropyl-7-(1S,6S-2,8-diazabicyclo[4.3.0]nonan-8-yl)-6-fluor-1,4-dihydro-4-oxo-3-chinolincarbonsäure, Verfahren zu seiner Herstellung sowie dieses enthaltende antibakterielle Mittel. 8-Cyan-1-cyclopropyl-7-(1S,6S-2,8-diazabicyclo[4.3.0]nonan-8-yl)-6-fluor-1,4-dihydro-4-oxo-3-chinolincarbonsäure der Formel (I) soll im folgenden als CCDC bezeichnet werden. 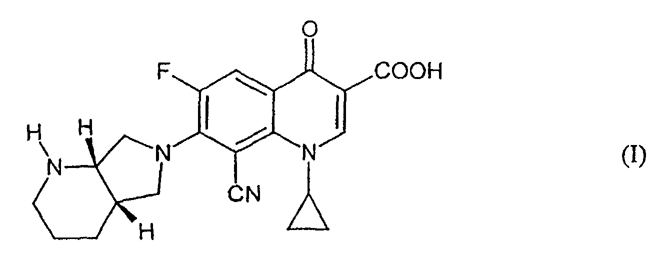 CCDC ist bekannt aus DE-A 19 633 805 oder PCT Anm.-Nr. 97 903 260.4 Sie wird hergestellt durch Umsetzung von 7-Halogen-8-cyan-1-cyclopropyl-6-fluor-1,4-dihydro-4-oxo-3-chinolincarbonsäure der Formel (II) 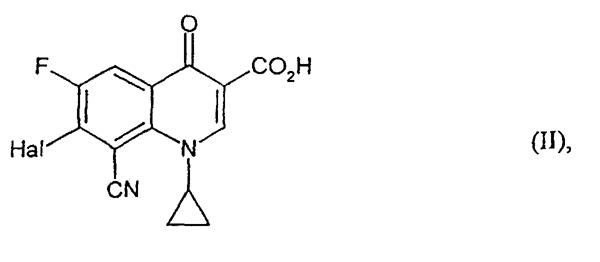 in welcher
- Hal: für Fluor oder bevorzugt für Chlor steht,
mit
(1S,6S)-2,8-Diazabicyclo[4.3.0]nonan der Formel (III) 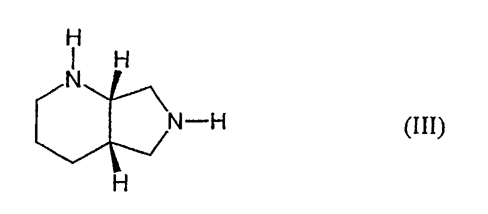 in Gegenwart einer Hilfsbase in einem geeigneten Lösungsmittel.

Mit CCDC der Formel (I) lassen sich ca. 0,02 %ige (w/w) Lösungen in Wasser herstellen. Diese Löslichkeit ist für praktische Anwendungen (Lösungen für Injektion oder orale Darreichungsformen) nicht ausreichend. Von vielen anderen Chinoloncarbonsäuren ist bekannt, daß sie in Form bestimmter Salze für Formulierungen eingesetzt werden. Als Salze kommen dabei einerseits Metallsalze der Chinoloncarbonsäure (z.B. Alkalicarboxylate) in Betracht, andererseits Säureadditionsprodukte (Protonierung basischer Zentren im Aminrest der substituierten Chinoloncarbonsäure). Als solche Säureadditionsprodukte werden häufig zum Beispiel die Mesylate, Tosylate und Hydrochloride verwendet. Die Hydrochloride lassen sich besonders einfach herstellen, sind pharmazeutisch akzeptabel und besitzen deutlich bessere Löslichkeiten als die Neutralverbindungen.

Das CCDC-Hydrochlorid der Formel (IV) 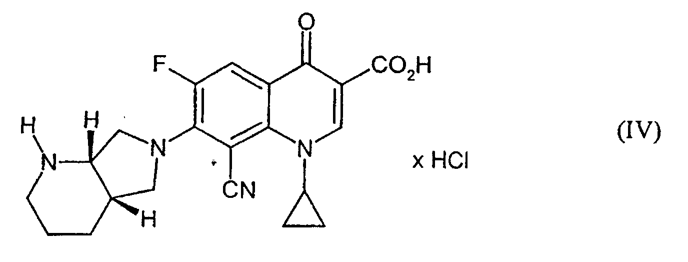 ist bekannt aus WO 97/31001.

Es läßt sich charakterisieren durch sein Röntgen-Pulverdiffraktogramm, die Differentialthermoanalyse (DTA) und sein Infrarot-Spektrum (IR).

Das Röntgen-Pulverdiffraktogramm zeigt die in der Tabelle 1 angegebenen Reflexlagen (2 Theta) hoher und mittlerer Intensität (> 30 % relative Intensität).

**Tabelle 1:**

| Pulver-Röntgendiffraktogramm von CCDC-Hydrochlorid der Formel (IV) |
|---|
| 2 θ (2 Theta) |
| 6,70 |
| 13,11 |
| 15,63 |
| 25,69 |
| 25,90 |

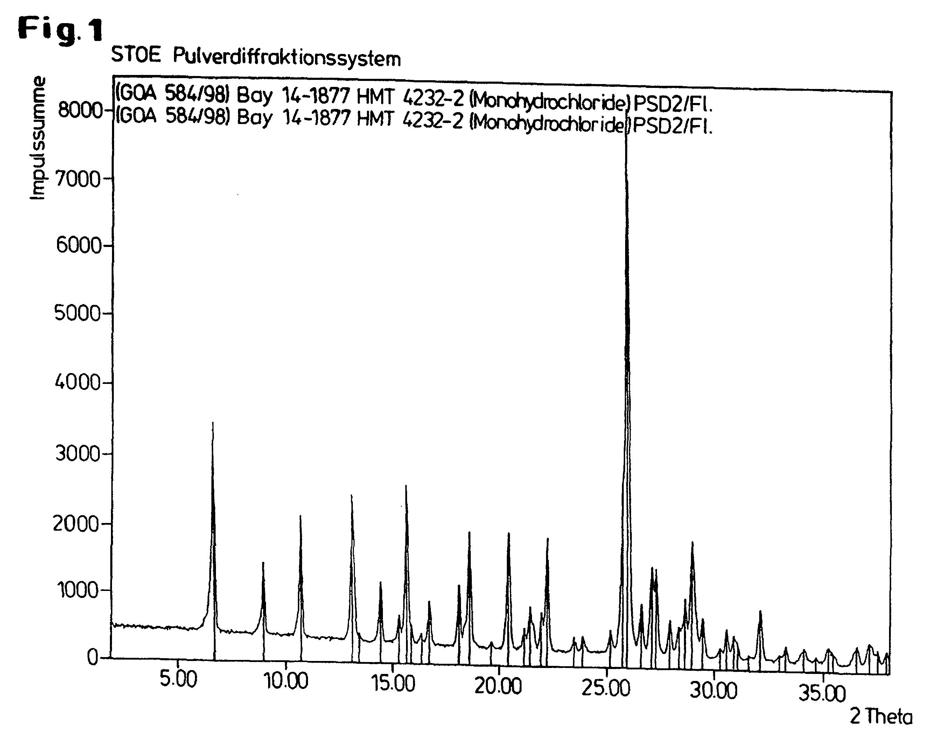

Das Pulver-Röntgendiffraktogramm von CCDC-Hydrochlorid der Formel (IV) ist auch in der Abbildung 1 wiedergegeben.

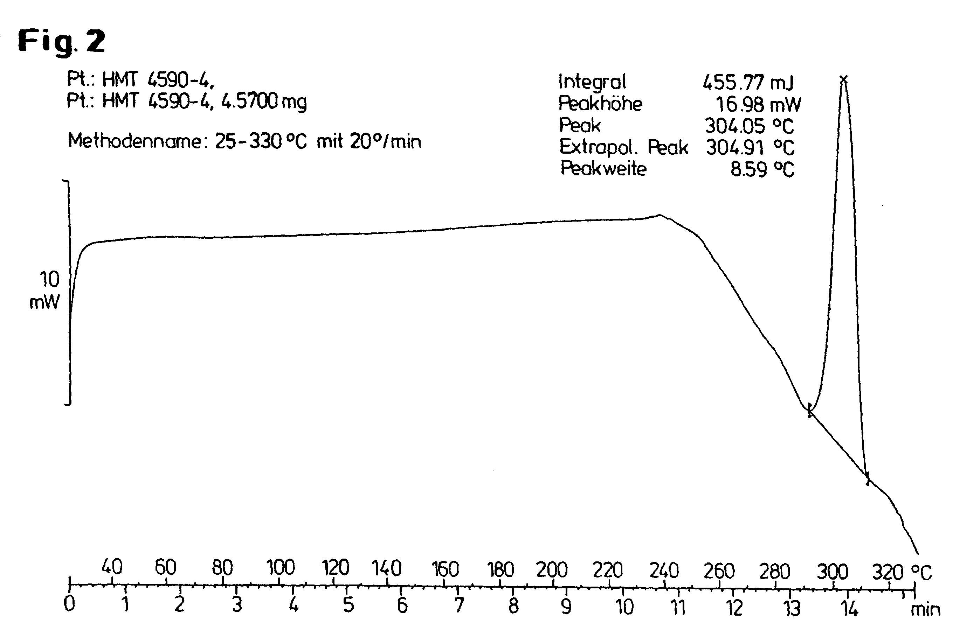

Der mit Hilfe der DTA bestimmte Schmelzpunkt des CCDC-Hydrochlorids der Formel (IV) beträgt 305°C bis 307°C (unter Zersetzung). Das Differentialthermodiagramm ist in der Abbildung 2 gezeigt.

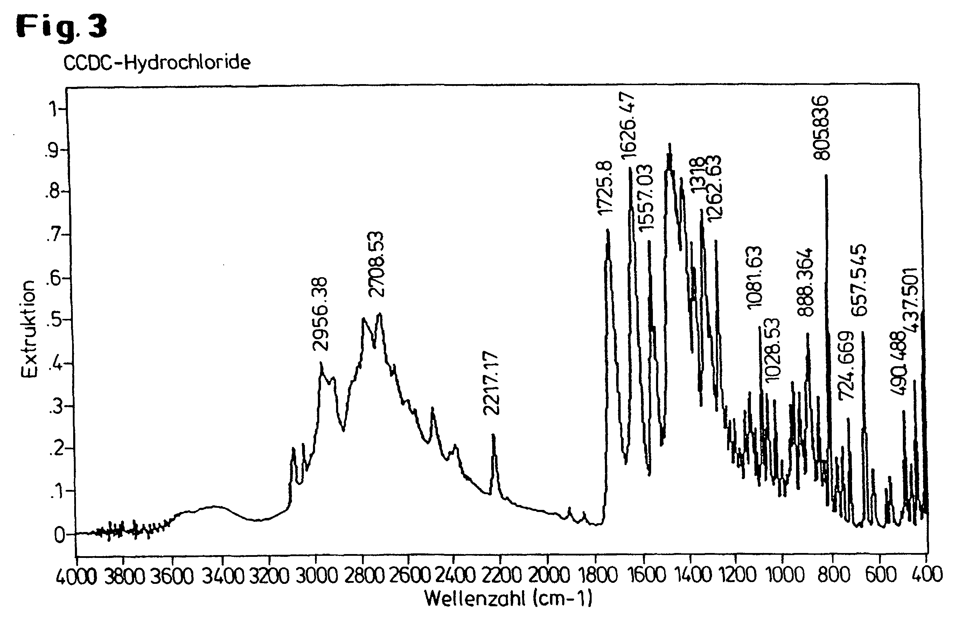

Das IR-Spektrum von CCDC-Hydrochlorid der Formel (IV) ist in der Abb. 3 wiedergegeben.

CCDC-Hydrochlorid der Formel (IV) kann nach im Prinzip bekannten Methoden hergestellt werden. So ist es z.B. möglich, eine Lösung von CCDC der Formel (I) in Wasser mit einem Moläquivalent HCl zu versetzen und die Lösung zur Trockene einzudampfen. Eine andere Methode besteht darin, 8-Cyan-1-cyclopropyl-7-(1S,6S-2,8-diazabicyclo[4.3.0]nonan-8-yl)-6-fluor-1,4-dihydro-4-oxo-3-chinolincarbonsäure-ethylester der Formel (V) 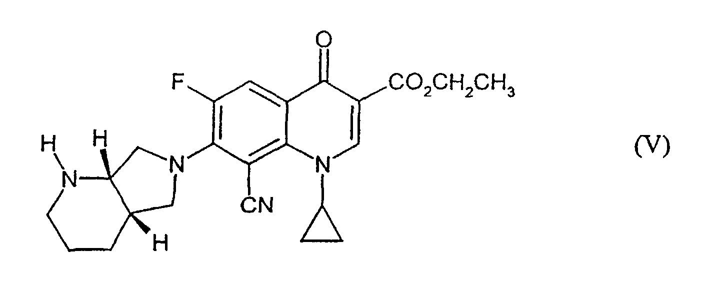 in wäßriger Salzsäure zu verseifen und das ausgefallenen CCDC-Hydrochlorid der Formel (IV) zu isolieren.

Mit CCDC-Hydrochlorid der Formel (IV) läßt sich eine ca. 2,8 %ige (w/w) Lösung in Wasser herstellen. Damit ist es zwar besser in Wasser löslich als CCDC der Formel (I), aber nicht in einer für alle Formulierungen wünschenswerten Menge.

Erfindungsgemäß wurde gefunden, daß überraschenderweise das CCDC-Semihydrochlorid der Formel (VI) 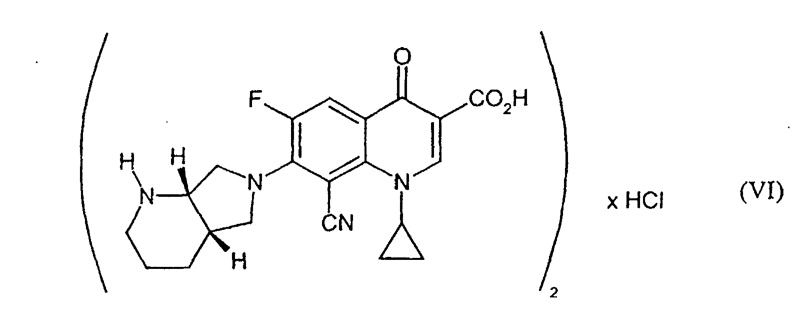 besonders gut in Wasser löslich ist. Mit CCDC-Semihydrochlorid der Formel (VI) konnen 19 %ige (w/w) Lösungen in Wasser hergestellt werden.

Gegenstand der Erfindung ist daher kristallines CCDC-Semihydrochlorid der Formel (VI), das unter anderem dadurch gekennzeichnet ist, daß es ein Röntgen-Pulverdiffraktogramm mit den in der Tabelle 2 angegebene Reflexlagen hoher und mittlerer Intensität (> 30 % relative Intensität) aufweist.

**Tabelle 2:**

| Pulver-Röntgendiffraktogramm von CCDC-Semihydrochlorid⁻ der Formel (VI) |
|---|
| 2 θ (2 Theta) |
| 5,86 |
| 6,90 |
| 7,26 |
| 8,98 |
| 9,35 |
| 10,13 |
| 10,68 |
| 10,97 |
| 12,41 |
| 13,67 |
| 14,57 |
| 14,89 |
| 15,73 |
| 16,07 |
| 16,47 |
| 16,87 |
| 17,78 |
| 18,91 |
| 19,81 |
| 20,04 |
| 20,62 |
| 20,75 |
| 20,93 |
| 21,46 |
| 21,74 |
| 22,92 |
| 25,36 |
| 25,71 |
| 26,98 |
| 27,58 |
| 28,24 |
| 30,61 |

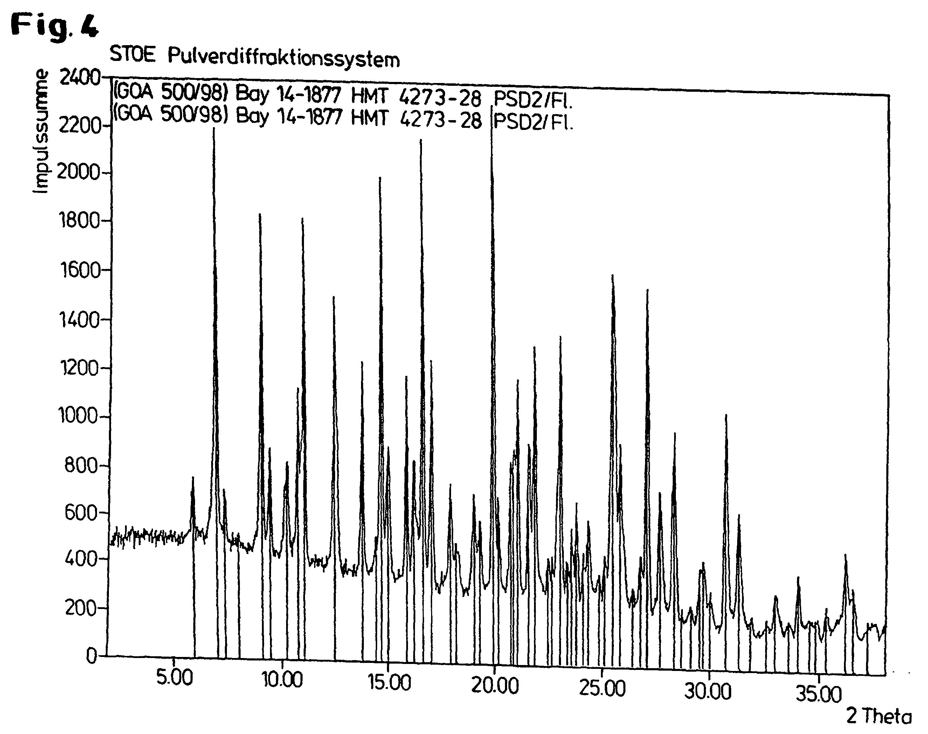

Das Pulver-Röntgendiffraktogramm des CCDC-Semihydrochlorids der Formel (VI) ist in der Abbildung 4 wiedergegeben.

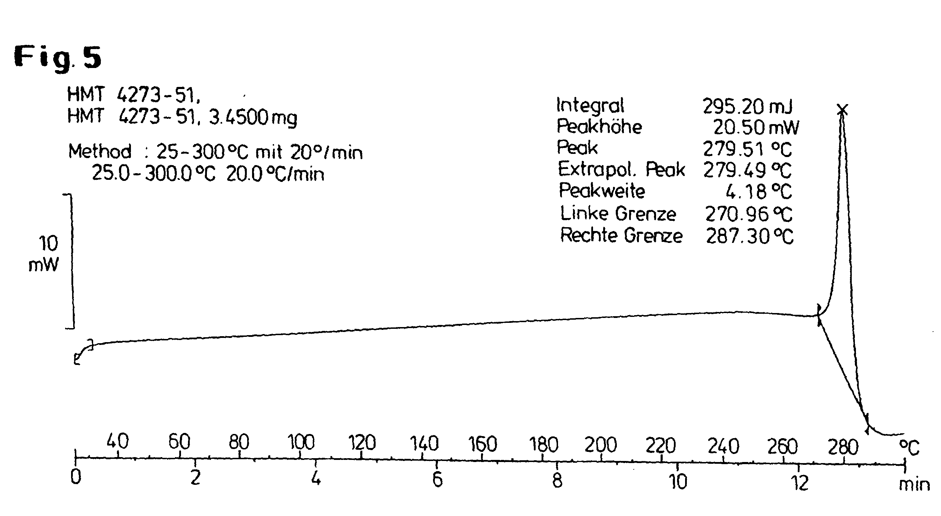

Das erfindungsgemäße CCDC-Semihydrochlorid der Formel (VI) ist weiterhin dadurch gekennzeichnet, daß es einen durch Differentialthermoanalyse bestimmten Schmelzpunkt von 278°C bis 280°C hat. Das entsprechende Differentialthermodiagramm zeigt die Abbildung 5.

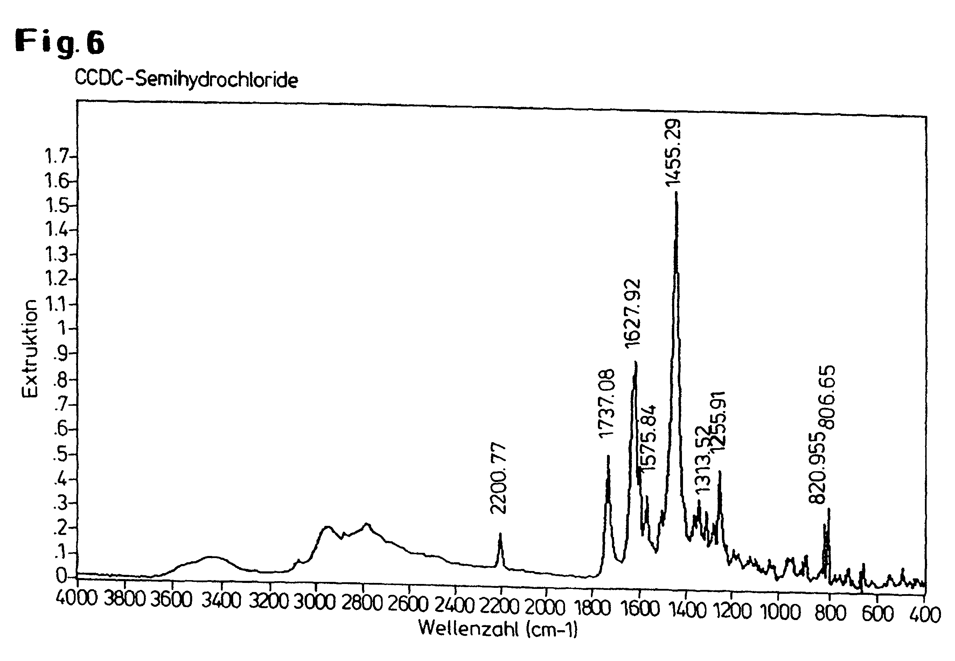

Das erfindungsgemäße CCDC-Semihydrochlorid der Formel (VI) ist weiterhin dadurch gekennzeichnet, daß es ein in KBr gemessenes Infrarotspektrum wie in Abbildung 6 gezeigt besitzt.

CCDC-Semihydrochlorid der Formel (VI) unbestimmter Kristallform kann nach im Prinzip bekannten Verfahren z.B. dadurch hergestellt werden, daß eine Lösung von CCDC der Formel (I) in Wasser mit einem halben Moläquivalent HCI versetzt wird und man die Lösung zur Trockene eindampft.

Ebenso ist es prinzipiell möglich, solche Mengen CCDC der Formel (I) und CCDC-Hydrochlorid der Formel (IV) im molaren Verhältnis eins zu eins in Wasser zu verrühren, daß die Gesamtkonzentration kleiner 20 % bleibt. Die erhaltene Lösung kann anschließend zur Trockene eingedampft werden.

Zudem wurde erfindungsgemäß gefunden, daß überraschenderweise ein CCDC-Semihydrochlorid der Formel (VI), das durch das oben angegebene Pulver-Röntgendiffraktogramm und das oben angegebene Differentialthermodiagramm charakterisiert ist, direkt hergestellt werden kann.

Gegenstand der vorliegenden Erfindung ist somit weiterhin das CCDC-Semihydrochlorid der Formel (VI), das dadurch gekennzeichnet ist, daß man 7-Halogen-8-cyan-1-cyclopropyl-6-fluor-1,4-dihydro-4-oxo-3-chinoloncarbonsäure der Formel (II), in welcher Halogen für Fluor oder bevorzugt für Chlor steht, mit (1S,6S)-2,8-Diazabicyclo[4.3.0]nonan der Formel (III) gegebenenfalls in Gegenwart einer Base in einem der folgenden Verdünnungsmittel oder Verdünnungsmittelgemische umsetzt:
a) aliphatische Alkohole mit mindestens vier Kohlenstoffatomen wie z.B. Butanol, Isobutanol, 2-Butanol, Tertiärbutanol, 1-Pentanol.
b) Gemisch von aliphatischen Alkoholen mit mindestens drei Kohlenstoffatomen wie z.B. Propanol, Isopropanol, Butanol, Isobutanol, 2-Butanol, Tertiärbutanol oder 1-Pentanol mit dem polar aprotischen Lösungsmittel N-Methyl-pyrrolidon.
c) Gemisch aus Propanol und N,N-Dimethylformamid, oder
d) Gemisch von Ethanol mit N-Methyl-pyrrolidon mit einem Basenzusatz an tertiären Aminen wie z.B. Tripropylamin, Tributylamin, N-Ethyl-morpholin, N-Propylmorpholin und/oder N-Butylmorpholin.

Bei den bevorzugten Herstellungsvarianten mit einem Verdünnungsmittelgemisch nach b) oder c) beträgt das Mischungsverhältnis 1:1 bis 3:1 und in besonders bevorzugten Ausführungsformen 1:1 bis 2:1.

Geeignete Basen sind gemäß den Herstellungsvarianten mit den Verdünnungsmitteln und Verdünnungsmittelgemischen nach a) bis c) tertiäre Amine wie z.B. Triethylamin, Tripropylamin, Ethyl-diisopropylamin (Hünig-Base), Tributylamin, N-Ethylmorpholin, N-Propylmorpholin und N-Butylmorpholin.

Bei den Herstellungsvarianten a) bis d) sind die Ausführungsformen bevorzugt, bei denen ein Überschuß an (1S,6S)-2,8-Diazabicyclo[4.3.0]nonan der Formel (III) eingesetzt wird.

Bei den Herstellungsvarianten nach a) bis d) werden auf 1 Mol der Verbindung (II) vorzugsweise 1 bis 2 Mol Base, besonders bevorzugt 1,1 bis 1,5 Mol Base, eingesetzt.

Die Umsetzung bei den Herstellungsvarianten nach a) bis d) erfolgt bei Normaldruck oder bei erhöhtem Druck zwischen 1 bar und 100 bar, vorzugsweise zwischen 1 bar und 20 bar.

Die Umsetzung bei den Herstellungsvarianten nach a) bis d) erfolgt bei Temperaturen zwischen 0°C und 200°C, vorzugsweise zwischen 20°C und 150°C.

Auf 1 Mol der Verbindung (II) werden vorzugsweise 1 bis 2 Mol, besonders bevorzugt 1 bis 1,5 Mol, der Verbindung (III) eingesetzt.

CCDC-Semihydrochlorid der Formel (VI) fällt aus der Reaktionsmischung aus und kann abgesaugt werden. Der abgesaugte Feststoff kann gegebenenfalls durch Waschen mit dem in der Reaktion verwendeten Alkohol gereinigt werden.

Die Ausgangsprodukte der Formeln (II) und (III) zur Herstellung von CCDC sind bekannt (vgl. DE-A 19 633 805).

CCDC-Semihydrochlorid der Formel (VI) ist hervorragend gegen pathogene Bakterien auf dem Gebiet der Human- oder Tiermedizin wirksam. Sein breites Einsatzgebiet entspricht dem von CCDC.

Das Röntgen-Pulverdiffraktogramm zur Charakterisierung der Kristallmodifikationen des CCDC Hydrochlorids und CCDC Semihydrochlorids wurde mit einem Transmissions-Diffraktometer STADI-P mit ortsempfindlichem Detektor (PSD2) der Fa. Stoe erhalten.

Der Schmelzpunkt der Differentialthermoanalyse wurde mit dem Gerät DSC 820 der Fa. Mettler-Toledo erhalten. Dabei wurde die Probe in einem Aluminiumtiegel mit 20 K/min an der Luft aufgeheizt.

Das IR-Spektrum wurde mit dem Gerät FTS 60 A der Fa. Biorad in KBr erhalten.

Die folgenden Beispiele illustrieren die Erfindung ohne sie einzuschränken. Die in den folgenden Beispielen beschriebenen Verdünnungsmittel/Basensysteme sind besonders bevorzugt.

### Vergleichsbeispiel

### Herstellung von CCDC-Hydrochlorid der Formel (IV)

850 g 8-Cyan-1-cyclopropyl-7-(1S,6S-2,8-diazabicyclo[4.3.0]nonan-8-yl)-6-fluor-1,4-dihydro-4-oxo-3-chinolincarbonsäure-ethylester werden in einer Mischung aus 1600 ml Wasser und 800 ml 10 %iger Salzsäure vorgelegt. Man erwärmt das Reaktionsgemisch bis zum Sieden, wobei der Ester in Lösung geht und bald darauf das Produkt auszufallen beginnt. Die Suspension wird für 3 Stunden zum Sieden erhitzt. Dann läßt man auf etwa 50°C abkühlen und gibt 1500 ml Ethanol hinzu. Das Reaktionsgemisch wird auf 0°C gekühlt und 1 Stunde bei dieser Temperatur gerührt. Man saugt den Feststoff ab, wäscht ihn mit 1000 ml Ethanol nach und trocknet bei 60°C bis zur Gewichtskonstanz. Es werden 845,5 g beiger Feststoff erhalten.

| Elementaranalyse (berechnete Werte für das Hydrochlorid C₂₁H₂₂ClFN₄O₃, Molekulargewicht 432,89): | |
|---|---|
| Kohlenstoff | gef. 58,2 % (ber. 58,27 %) |
| Wasserstoff | gef. 5,1 % (ber. 5,12 %) |
| Chlor | gef. 8,1 % (ber. 8,19 %). |

Das Produkt zeigt das in der Abbildung 1 wiedergegebene Röntgen-Pulverdiffraktogramm, das in der Abbildung 2 wiedergegebene Differentialthermodiagramm und das in der Abbildung 3 wiedergegebene IR-Spektrum.

### Herstellung von CCDC-Semihydrochlorid der Formel (VI)

### Beispiel 1

9,2 g 7-Chlor-8-cyan-1-cyclopropyl-6-fluor-1,4-dihydro-4-oxo-3-chinolincarbonsäure werden in einer Mischung aus 30 ml Butanol, 18 ml N-Methyl-pyrrolidon und 4,85 g Hünig-Base vorgelegt. Man erhitzt zum Rückfluß und tropft dann 4,17 g 1S,6S)-2,8-Diazabicyclo[4.3.0]nonan zu. Nach Beendigung des Zutropfens rührt man noch 3 Stunden unter Rückfluß, läßt dann auf Raumtemperatur abkühlen, saugt den Feststoff ab, wäscht ihn mit insgesamt 20 ml Butanol und trocknet bei 60 bis 70°C im Vakuumtrockenschrank bis zur Gewichtskonstanz.

Man erhält 8,54 g beigen Feststoff, der das in der Abbildung 4 gezeigte Röntgen-Pulverdiffraktogramm und das in der Abbildung 5 gezeigte Differentialthermodiagramm aufweist.

Elementaranalyse (berechnete Werte für das CCDC-Semihydrochlorid C₂₁H_{22,5}Cl_{0,5}FN₄O₃, Molekulargewicht 414,658):
Chlor: gef. 4,2% (ber. 4,275%).

### Beispiel 2

Eine Mischung aus 9,2 g 7-Chlor-8-cyan-1-cyclopropyl-6-fluor-1,4-dihydro-4-oxo-3-chinolincarbonsäure, 60 ml Butanol und 4,85 g Hünig-Base wird zum Rückfluß erhitzt. Man tropft 4,17 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 20 ml Butanol gewaschen und bis zur Gewichtskonstanz getrocknet. Man erhält 10,6 g beigen Feststoff, dessen Differentialthermodiagramm dem des CCDC-Semihydrochlorids der Formel (VI) entspricht.

| Elementaranalyse (berechnete Werte für das CCDC-Semihydrochlorid C₂₁H_{22,5}Cl_{0,5}FN₄O₃, Molekulargewicht 414,658): | |
|---|---|
| Kohlenstoff | gef. 60,55 % (ber. 60,83 %) |
| Wasserstoff | gef. 5,3 % (ber. 5,23 %) |
| Chlor | gef. 4,2 % (ber. 4,275 %) |
| Stickstoff | gef. 13,5 % (ber. 13,51 %) |
| Sauerstoff | gef. 11,7 % (ber. 11,58 %). |

### Beispiel 3

Eine Mischung aus 4,6 g 7-Chlor-8-cyan-1-cyclopropyl-6-fluor-1,4-dihydro-4-oxo-3-chinolincarbonsäure, 15 ml Propanol, 9 ml N-Methyl-pyrrolidon und 2,42 g Hünig-Base wird zum Rückfluß erhitzt. Man tropft 2,08 g (1S,6S)-2,8-Diazabicyclo-[4.3.0]nonan zu und rührt dann 3 Stunden bei Rückfluß. Der Feststoff wird bei Raumtemperatur abgesaugt, mit insgesamt 10 ml Propanol gewaschen und bis zur Gewichtskonstanz getrocknet. Man erhält 4,6 g beigen Feststoff dessen Differentialthermodiagramm dem des CCDC-Semihydrochlorids entspricht.

Elementaranalyse (berechnete Werte für das CCDC-Semihydrochlorid C₂₁H_{22,5}Cl_{0,5}FN₄O₃, Molekulargewicht 414,658):
Chlor: gef. 4,3 % (ber. 4,275 %).

### Beispiel 4

Eine Mischung aus 4,6 g 7-Chlor-8-cyan-1-cyclopropyl-6-fluor-1,4-dihydro-4-oxo-3-chinolincarbonsäure, 15 ml Isopropanol, 9 ml N-Methyl-pyrrolidon und 2,42 g Hünig-Base wird zum Rückfluß erhitzt. Man tropft 2,08 g (1S,6S)-2,8-Diazabicyclo-[4.3.0]nonan zu und rührt dann 3 Stunden bei Rückfluß. Der Feststoff wird bei Raumtemperatur abgesaugt, mit insgesamt 10 ml Isopropanol gewaschen und bis zur Gewichtskonstanz getrocknet. Man erhält 5,3 g beigen Feststoff.

Elementaranalyse (berechnete Werte für das CCDC-Semihydrochlorid C₂₁H_{22,5}Cl_{0,5}FN₄O₃, Molekulargewicht 414,658):
Chlor: gef. 4,2 % (ber. 4,275 %).

### Beispiel 5

Eine Mischung aus 4,6 g 7-Chlor-8-cyan-1-cyclopropyl-6-fluor-1,4-dihydro-4-oxo-3-chinolincarbonsäure, 15 ml 2-Butanol, 9 ml N-Methyl-pyrrolidin und 2,42 g Hünig-Base wird zum Rückfluß erhitzt. Man tropft 2,08 g (1S,6S)-2,8-Diazabicyclo-[4.3.0]nonan zu und rührt dann 3 Stunden bei Rückfluß. Der Feststoff wird bei Raumtemperatur abgesaugt, mit insgesamt 10 ml 2-Butanol gewaschen und bis zur Gewichtskonstanz getrocknet. Man erhält 5,48 g beigen Feststoff, dessen Differentialthermodiagramm dem des CCDC-Semihydrochlorids der Formel (VI) entspricht.

Elementaranalyse (berechnete Werte für das CCDC-Semihydrochlorid C₂₁H_{22,5}Cl_{0,5}FN₄O₃, Molekulargewicht 414,658):
Chlor: gef. 4,2 % (ber. 4,275 %).

### Beispiel 6

Eine Mischung aus 4,6 g 7-Chlor-8-cyan-1-cyclopropyl-6-fluor-1,4-dihydro-4-oxo-3-chinolincarbonsäure, 15 ml Isobutanol, 9 ml N-Methyl-pyrrolidin und 2,42 g Hünig-Base wird zum Rückfluß erhitzt. Man tropft 2,08 g (1S,6S)-2,8-Diazabicyclo[4.3.0]-nonan zu und rührt dann 3 Stunden bei Rückfluß. Der Feststoff wird bei Raumtemperatur abgesaugt, mit insgesamt 10 ml Isobutanol gewaschen und bis zur Gewichtskonstanz getrocknet. Man erhält 4,99 g beigen Feststoff, dessen Differentialthermodiagramm dem des CCDC-Semihydrochlorids der Formel (VI) entspricht.

Elementaranalyse (berechnete Werte für das CCDC-Semihydrochlorid C₂₁H_{22,5}Cl_{0,5}FN₄O₃, Molekulargewicht 414,658):
Chlor: gef. 4,2 % (ber. 4,275 %).

### Beispiel 7

Eine Mischung aus 4,6 g 7-Chlor-8-cyan-1-cyclopropyl-6-fluor-1,4-dihydro-4-oxo-3-chinolincarbonsäure, 15 ml Tertiärbutanol, 9 ml N-Methyl-pyrrolidin und 2,42 g Hünig-Base wird zum Rückfluß erhitzt. Man tropft 2,08 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 10 ml erwärmten Tertiärbutanol gewaschen und bis zur Gewichtskonstanz getrocknet. Man erhält 5,38 g beigen Feststoff, dessen Differentialthermodiagramm dem des CCDC-Semihydrochlorids der Formel (VI) entspricht.

Elementaranalyse (berechnete Werte ir das CCDC-Semihydrochlorid C₂₁H_{22,5}Cl_{0,5}FN₄O₃, Molekulargewicht 414,658):
Chlor: gef. 4,2 % (ber. 4,275 %).

### Beispiel 8

Eine Mischung aus 4,6 g 7-Chlor-8-cyan-1-cyclopropyl-6-fluor-1,4-dihydro-4-oxo-3-chinolincarbonsäure, 15 ml 1-Pentanol, 9 ml N-Methyl-pyrrolidin und 2,42 g Hünig-Base wird zum Rückfluß erhitzt. Man tropft 2,08 g (1S,6S)-2,8-Diazabicyclo[4.3.0]-nonan zu und rührt dann 3 Stunden bei Rückfluß. Der Feststoff wird bei Raumtemperatur abgesaugt, mit insgesamt 10 ml 1-Pentanol gewaschen und bis zur Gewichtskonstanz getrocknet. Man erhält 3,0 g beigen Feststoff, dessen Differentialthermodiagramm dem des CCDC-Semihydrochlorids der Formel (VI) entspricht.

Elementaranalyse (berechnete Werte für das CCDC-Semihydrochlorid C₂₁H_{22,5}Cl_{0,5}FN₄O₃, Molekulargewicht 414,658):
Chlor: gef. 4,3 % (ber. 4,275 %).

### Beispiel 9

Eine Mischung aus 4,6 g 7-Chlor-8-cyan-1-cyclopropyl-6-fluor-1,4-dihydro-4-oxo-3-chinolincarbonsäure, 15 ml Ethanol, 9 ml N-Methyl-pyrrolidin und 3,47 g Tributylamin wird zum Rückfluß erhitzt. Man tropft 2,08 g (1S,6S)-2,8-Diazabicyclo-[4.3.0]nonan zu und rührt dann 3 Stunden bei Rückfluß. Der Feststoff wird bei Raumtemperatur abgesaugt, mit insgesamt 10 ml Ethanol gewaschen und bis zur Gewichtskonstanz getrocknet. Man erhält 5,0 g beigen Feststoff, dessen Differentialthermodiagramm dem des CCDC-Semihydrochlorids der Formel (VI) entspricht.

Elementaranalyse (berechnete Werte für das CCDC-Semihydrochlorid C₂₁H_{22,5}Cl_{0,5}FN₄O₃, Molekulargewicht 414,658):
Chlor: gef. 4,2 % (ber. 4,275 %).

### Beispiel 10

Eine Mischung aus 4,6 g 7-Chlor-8-cyan-1-cyclopropyl-6-fluor-1,4-dihydro-4-oxo-3-chinolincarbonsäure, 15 ml Ethanol, 9 ml N-Methyl-pyrrolidin und 2,16 g N-Ethylmorpholin wird zum Rückfluß erhitzt. Man tropft 2,08 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 10 ml Ethanol gewaschen und bis zur Gewichtskonstanz getrocknet. Man erhält 5,4 g beigen Feststoff.

Elementaranalyse (berechnete Werte für das CCDC-Semihydrochlorid C₂₁H_{22,5}Cl_{0,5}FN₄O₃, Molekulargewicht 414,658):
Chlor: gef. 4,3 % (ber. 4,275 %).

### Beispiel 11

Eine Mischung aus 4,6 g 7-Chlor-8-cyan-1-cyclopropyl-6-fluor-1,4-dihydro-4-oxo-3-chinolincarbonsäure, 15 ml Propanol, 9 ml N,N-Dimethylformamid und 2,42 g Hünig-Base wird zum Rückfluß erhitzt. Man tropft 2,08 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 10 ml Propanol gewaschen und bis zur Gewichtskonstanz getrocknet. Man erhält 4,4 g beigen Feststoff.

Elementaranalyse (berechnete Werte für das CCDC-Semihydrochlorid C₂₁H_{22,5}Cl_{0,5}FN₄O₃, Molekulargewicht 414,658):
Chlor: gef. 4,2 % (ber. 4,275 %).

## Patentansprüche

1. Semi-Hydrochlorid von 8-Cyan-1-cyclopropyl-7-(1S,6S-2,8-diazabicyclo-[4.3.0]nonan-8-yl)-6-fluor-1,4-dihydro-4-oxo-3-chinolincarbonsäure.

2. Semi-Hydrochlorid von 8-Cyan-1-cyclopropyl-7-(1S,6S-2,8-diazabicyclo-[4.3.0]nonan-8-yl)-6-fluor-1,4-dihydro-4-oxo-3-chinolincarbonsäure (CCDC-Semihydrochlorid) gemäß Anspruch 1, **dadurch gekennzeichnet, dass** es ein Röntgen-Pulverdiffraktogramm mit folgenden Reflexlagen (2 Theta) hoher und mittlerer Intensität (>30 % relative Intensität) aufweist:
| 2 θ ( 2 Theta ) |
|---|
| 5,86 |
| 6,90 |
| 7,26 |
| 8,98 |
| 9,35 |
| 10,13 |
| 10,68 |
| 10,97 |
| 12,41 |
| 13,67 |
| 14,57 |
| 14,89 |
| 15,73 |
| 16,07 |
| 16,47 |
| 16,87 |
| 17,78 |
| 18,91 |
| 19,81 |
| 20,04 |
| 20,62 |
| 20,75 |
| 20,93 |
| 21,46 |
| 21,74 |
| 22,92 |
| 25,36 |
| 25,71 |
| 26,98 |
| 27,58 |
| 28,24 |
| 30,61 |

3. Semi-Hydrochlorid von 8-Cyan-1-cyclopropyl-7-(lS,6S-2,8-diazabicyclo-[4.3.0]nonan-8-yl)-6-fluor-1,4-dihydro-4-oxo-3-chinolincarbonsäure (CCDC-Semihydrochlorid), **dadurch gekennzeichnet, dass** es ein Röntgen-Pulverdiffraktogramm mit folgenden Reflexlagen (2 Theta) hoher und mittlerer Intensität (>30 % relative Intensität) aufweist:
| 2θ(2 Theta) |
|---|
| 5,86 |
| 6,90 |
| 7,26 |
| 8,98 |
| 9,35 |
| 10,13 |
| 10,68 |
| 10,97 |
| 12,41 |
| 13,67 |
| 14,57 |
| 14,89 |
| 15,73 |
| 16,07 |
| 16,47 |
| 16,87 |
| 17,78 |
| 18,91 |
| 19,81 |
| 20,04 |
| 20,62 |
| 20,75 |
| 20,93 |
| 21,46 |
| 21,74 |
| 22,92 |
| 25,36 |
| 25,71 |
| 26,98 |
| 27,58 |
| 28,24 |
| 30,61 |
und einen durch DTA ermittelten Schmelzpunkt von 278°C bis 280°C hat.

4. CCDC-Semihydrochlorid gemäß Anspruch 1 oder 2, dadurch erhältlich, dass 7-Halogen-8-cyan-1-cyclopropyl-6-fluor-1,4-dihydro-4-oxo-3-chinolincarbonsäure der Formel (II) 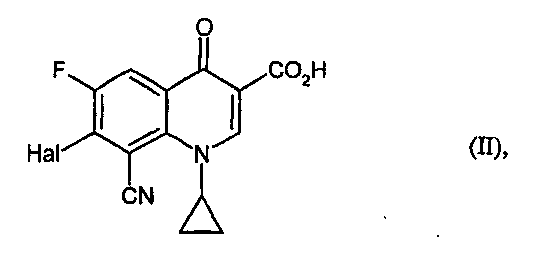 in welcher
Hal für Chlor steht,
und (1S,6S-2,8-Diazabicyclo[4.3.0]nonan der Formel (III) 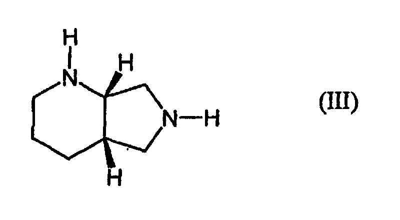 gegebenenfalls in Gegenwart einer Base in einem der folgenden Verdünnungsmittel oder Verdünnungsmittelgemische umgesetzt werden:
a) aliphatische Alkohole mit mindestens vier Kohlenstoffatomen,
b) Gemisch von aliphatischen Alkoholen mit mindestens drei Kohlenstoffatomen mit dem Verdünnungsmittel N-Methyl-pyrrolidon,
c) Gemisch aus Propanol und N,N-Dimethylformamid,
oder
d) Gemisch von Ethanol mit N-Methyl-pyrrolidon mit einem Basenzusatz an Tripropylamin, Tributylamin, N-Ethylmorpholin, N-Propylmorpholin und/oder N-Butylmorpholin.

5. Verfahren zur Herstellung von CCDC-Semihydrochlorid gemäß einem der Ansprüche 1 bis 4, **dadurch gekennzeichnet, dass** 7-Halogen-8-cyan-1-cyclopropyl-6-fluor-1,4-dihydro-4-oxo-3-chinolincarbonsäure der Formel (II) 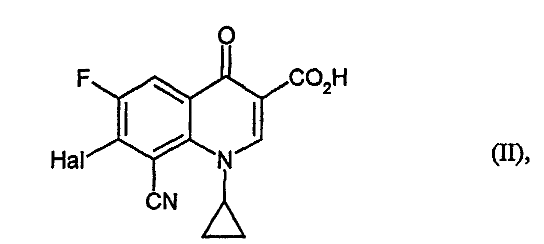 in welcher
Hal für Chlor steht,
und (1S,6S-2,8-Diazabicyclo[4.3.0]nonan der Formel (III) 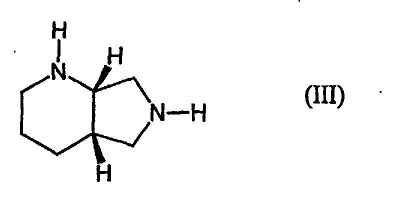 gegebenenfalls in Gegenwart einer Base in einem der folgenden Verdünnungsmittel oder Verdünnungsmittelgemisch umgesetzt werden:
a) aliphatische Alkohole mit mindestens vier Kohlenstoffatomen,
b) Gemisch von aliphatischen Alkoholen mit mindestens drei Kohlenstoffatomen mit dem Verdünnungsmittel N-Methyl-pyrrolidon,
c) Gemisch aus Propanol und N,N-Dimethylformamid,
oder
d) Gemisch von Ethanol mit N-Methyl-pyrrolidon mit einem Basenzusatz an Tripropylamin, Tributylamin, N-Ethylmorpholin, N-Propylmorpholin und/oder N-Butylmorpholin.

6. Verfahren zur Herstellung von CCDC-Semihydrochlorid gemäß Anspruch 5, **dadurch gekennzeichnet, dass** als Verdünnungsmittel ein aliphatischer Alkohol mit mindestens 4 Kohlenstoffatomen oder als Bestandteil eines Verdünnungsmittelgemisches ein aliphatischer Alkohol mit mindestens 2 Kohlenstoffatomen eingesetzt wird.

7. Verfahren zur Herstellung von CCDC-Semihydrochlorid gemäß Anspruch 5, **dadurch gekennzeichnet, dass** bei Verwendung eines aliphatischen Alkohols mit mindestens 3 Kohlenstoffatomen als Bestandteil eines Verdünnungsmittelgemisches gleichzeitig als weiteres Verdünnungsmittel N-Methyl-pyrrolidon im Verhältnis 1 zu 1 bis 3 zu 1 eingesetzt wird.

8. Verfahren zur Herstellung von CCDC-Semihydrochlorid gemäß Anspruch 6, **dadurch gekennzeichnet, dass** bei Verwendung von Propanol als Bestandteil eines Verdünnungsmittelgemisches gleichzeitig als weiteres Verdünnungsmittel N,N-Dimethylformamid im Verhältnis 1 zu 1 bis 3 zu 1 eingesetzt wird.

9. Arzneimittel, **dadurch gekennzeichnet, dass** es neben üblichen Hilfs- und Trägerstoffen CCDC-Semihydrochlorid gemäß einem der Ansprüche 1 bis 4 enthält.

10. Verwendung von CCDC-Semihydrochlorid gemäß einem der Ansprüche 1 bis 4 zur Herstellung von Arzneimitteln.

11. CCDC-Semihydrochlorid gemäß einem der Ansprüche 1 bis 4 zur Verwendung in antibakteriellen Mitteln.

## Claims

1. Semi-hydrochloride of 8-cyano-1-cyclopropyl-7-(1S,6S-2,8-diazabicyclo[4.3.0]nonan-8-yl)-6-fluoro-1,4-dihydro-4-oxo-3-quinolinecarboxylic acid.

2. Semi-hydrochloride of 8-cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC semihydrochloride) accordingg to Claim 1, **characterized in that** it has an X-ray powder diffractogram with the following reflection signals (2 theta) of high and medium intensity (> 30% relative intensity):
| 2 θ ( 2 Theta ) |
|---|
| 5.86 |
| 6.90 |
| 7.26 |
| 8.98 |
| 9.35 |
| 10.13 |
| 10.68 |
| 10.97 |
| 12.41 |
| 13.67 |
| 14.57 |
| 14.89 |
| 15.73 |
| 16.07 |
| 16.47 |
| 16.87 |
| 17.78 |
| 18.91 |
| 19.81 |
| 20.04 |
| 20.62 |
| 20.75 |
| 20.93 |
| 21.46 |
| 21.74 |
| 22.92 |
| 25.36 |
| 25.71 |
| 26.98 |
| 27.58 |
| 28.24 |
| 30.61 |

3. Semi-hydrochloride of 8-cyano-1-cyclopropyl-7-[1S,6S-2,8-diazabicyclo[4.3.0]nonan-8-yl)-6-fluoro-1,4-dihydro-4-oxo-3-quinolinecarboxylic acid (CCDC semihydrochloride), **characterized in that** it has an X-ray powder diffractogram with the following reflection signals (2 theta) of high and medium intensity (> 30% relative intensity):
| 2 θ ( 2 Theta ) |
|---|
| 5.86 |
| 6.90 |
| 7.26 |
| 8.98 |
| 9.35 |
| 10.13 |
| 10.68 |
| 10.97 |
| 12.41 |
| 13.67 |
| 14.57 |
| 14.89 |
| 15.73 |
| 16.07 |
| 16.47 |
| 16.87 |
| 17.78 |
| 18.91 |
| 19.81 |
| 20.04 |
| 20.62 |
| 20.75 |
| 20.93 |
| 21.46 |
| 21.74 |
| 22.92 |
| 25.36 |
| 25.71 |
| 26.98 |
| 27.58 |
| 28.24 |
| 30.61 |
and a melting point, determined by DTA, of from 278°C to 280°C.

4. CCDC semihydrochloride according to Claim 1 or 2, obtainable by reacting 7-halogeno-8-cyano-1-cyclopropyl-6-fluoro-1,4-dihydro-4-oxo-3-quinolinecarboxylic acid of the formula (II) 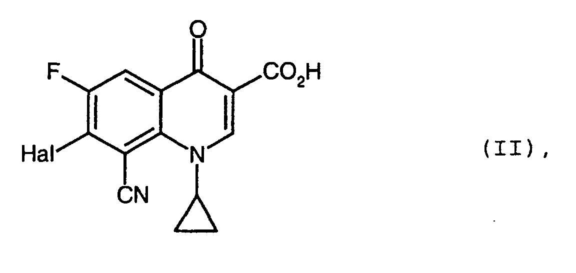 in which
Hal represents chlorine,
and (1S,6S-2,8-diazabicyclo[4.3.0]nonane of the formula (III) 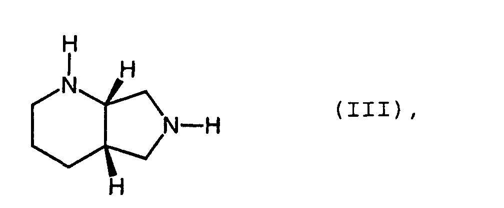 if appropriate in the presence of a base, in one of the following diluents or diluent mixtures:
a) aliphatic alcohols having at least four carbon atoms,
b) mixture of aliphatic alcohols having at least three carbon atoms with the diluent N-methylpyrrolidone,
c) mixture of propanol and N,N-dimethylformamide,
or
d) mixture of ethanol with N-methyl-pyrrolidone with added tripropylamine, tributylamine, N-ethylmorpholine, N-propylmorpholine and/or N-butylmorpholine base.

5. Process for preparing CCDC semihydrochloride according to any of Claims 1 to 4, **characterized in that** 7-halogeno-8-cyano-1-cyclopropyl-6-fluoro-1,4-dihydro-4-oxo-3-quinolinecarboxylic acid of the formula (II) 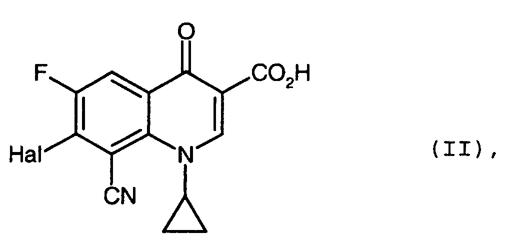 in which
Hal represents chlorine
and (1S,6S-2,8-diazabicyclo[4.3.0]nonane of the formula (III) 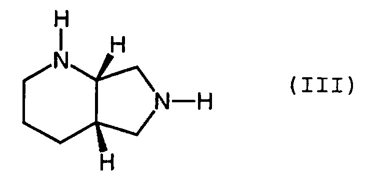 are reacted if appropriate in the presence of a base in one of the following diluents or diluent mixtures:
a) aliphatic alcohols having at least four carbon atoms,
b) mixture of aliphatic alcohols having at least three carbon atoms with the diluent N-methylpyrrolidone,
c) mixture of propanol and N,N-dimethylformamide,
or
d) mixture of ethanol with N-methyl-pyrrolidone with added tripropylamine, tributylamine, N-ethylmorpholine, N-propylmorpholine and/or N-butylmorpholine base.

6. Process for preparing CCDC semihydrochloride according to Claim 5, **characterized in that** the diluent used is an aliphatic alcohol having at least 4 carbon atoms or that an aliphatic alcohol having at least two carbon atoms is used as component of a diluent mixture.

7. Process for preparing CCDC semihydrochloride according to Claim 5, **characterized in that**, if an aliphatic alcohol having at least 3 carbon atoms is used as component of a diluent mixture, N-methyl-pyrrolidone is simultaneously employed as a further diluent in a ratio of from 1 to 1 to 3 to 1.

8. Process for preparing CCDC semihydrochloride according to Claim 6, **characterized in that**, if propanol is used as component of a diluent mixture, N,N-dimethylformamide is simultaneously employed as further diluent in a ratio of from 1 to 1 to 3 to 1.

9. Medicament, **characterized in that** it comprises, in addition to customary auxiliaries and excipients, CCDC semihydrochloride according to any of Claims 1 to 4.

10. Use of CCDC semihydrochloride according to any of Claims 1 to 4 for preparing medicaments.

11. CCDC semihydrochloride according to any of Claims 1 to 4 for use in antibacterial compositions.

## Revendications

1. Semichlorhydrate de l'acide 8-cyano-1-cyclopropyl-7-(1S,6S-2,8-diazabicyclo[4.3.0]nonane-8-yl)-6-fluoro-1,4-dihydro-4-oxo-3-quinoléine-carboxylique.

2. Semichlorhydrate de l'acide 8-cyano-1-cyclopropyl-7-(1S,6S-2,8-diazabicyclo[4.3.0]nonane-8-yl)-6-fluoro-1,4-dihydro-4-oxo-3-quinoléine-carboxylique (semichlorhydrate de CCDC) suivant la revendication 1, **caractérisé en ce que** son diffractogramme des rayons X sur poudre présente les positions de réflexion (2 thêta) de hautes et moyennes intensités (> 30 % d'intensité relative) suivantes :
| 2 θ (2 thêta) |
|---|
| 5,86 |
| 6,90 |
| 7,26 |
| 8,98 |
| 9,35 |
| 10,13 |
| 10,68 |
| 10,97 |
| 12,41 |
| 13,67 |
| 14,57 |
| 14,89 |
| 15,73 |
| 16,07 |
| 16,47 |
| 16,87 |
| 17,78 |
| 18,91 |
| 19,81 |
| 20,04 |
| 20,62 |
| 20,75 |
| 20,93 |
| 21,46 |
| 21,74 |
| 22,92 |
| 25,36 |
| 25,71 |
| 26,98 |
| 27,58 |
| 28,24 |
| 30,61 |

3. Semichlorhydrate de l'acide 8-cyano-1-cyclopropyl-7-(1S,6S-2,8-diazabicyclo[4.3.0]nonane-8-yl)-6-fluoro-1,4-dihydro-4-oxo-3-quinoléine-carboxylique (semichlorhydrate de CCDC), **caractérisé en ce que** son diffractogramme des rayons X sur poudre présente les positions de réflexion (2 thêta) de haute et moyenne intensité (> 30 % d'intensité relative) suivantes :
| 2 θ (2 thêta) |
|---|
| 5,86 |
| 6,90 |
| 7,26 |
| 8,98 |
| 9,35 |
| 10,13 |
| 10,68 |
| 10,97 |
| 12,41 |
| 13,67 |
| 14,57 |
| 14,89 |
| 15,73 |
| 16,07 |
| 16,47 |
| 16,87 |
| 17,78 |
| 18,91 |
| 19,81 |
| 20,04 |
| 20,62 |
| 20,75 |
| 20,93 |
| 21,46 |
| 21,74 |
| 22,92 |
| 25,36 |
| 25,71 |
| 26,98 |
| 27,58 |
| 28,24 |
| 30,61 |
et un point de fusion déterminé par analyse thermique différentielle de 278°C à 280°C.

4. Semichlorhydrate de CCDC suivant la revendication 1 ou 2, pouvant être obtenu par réaction d'un acide 7-halogéno-8-cyano-1-cyclopropyl-6-fluoro-1,4-dihydro-4-oxo-3-quinoléine-carboxylique de formule II 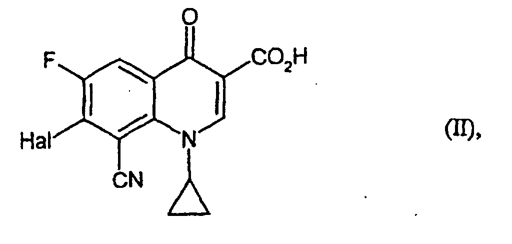 dans laquelle
Hal représente le chlore,
et du (1S,6S-2,8-diazabicyclo[4.3.0]nonane de formule III 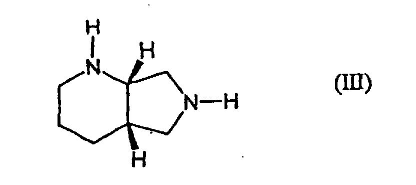 éventuellement en présence d'une base, dans l'un des diluants suivants ou de mélanges de ces diluants :
a) alcools aliphatiques ayant au moins 4 atomes de carbone,
b) mélange d'alcools aliphatiques ayant au moins trois atomes de carbone avec la N-méthylpyrrolidone comme diluant,
c) mélange de propanol et de N,N-diméthylformamide,
ou
d) mélange d'éthanol avec la N-méthylpyrrolidone additionné, comme base, de tripropylamine, de tributylamine, de N-éthylmorpholine, de N-propylmorpholine et/ou de N-butylmorpholine.

5. Procédé de production de semichlorhydrate de CCDC suivant l'une des revendications 1 à 4, **caractérisé en ce qu'**on fait réagir un acide 7-halogéno-8-cyano-1-cyclopropyl-6-fluoro-1,4-dihydroxy-4-oxo-3-quinoléinecarboxylique de formule II 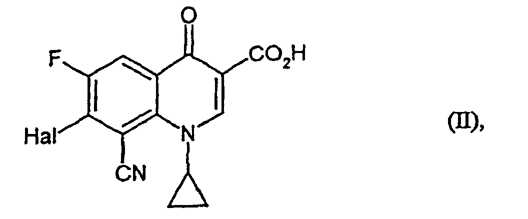 dans laquelle
Hal représente le chlore,
avec le (1S,6S-2,8-diazabicyclo[4.3.0]nonane de formule (III) 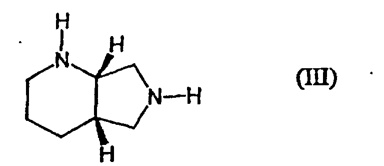 éventuellement en présence d'une base, dans l'un des diluants suivants ou un mélange de ces diluants :
a) alcools aliphatiques ayant au moins 4 atomes de carbone,
b) mélange d'alcools aliphatiques ayant au moins trois atomes de carbone avec la N-méthylpyrrolidone comme diluant,
c) mélange de propanol et de N,N-diméthylformamide,
ou
d) mélange d'éthanol et de N-méthylpyrrolidone additionné, comme base, de tripropylamine, de tributylamine, de N-éthylmorpholine, de N-propylmorpholine et/ou de N-butylmorpholine.

6. Procédé de production de semichlorhydrate de CCDC suivant la revendication 5, **caractérisé en ce qu'**on utilise comme diluant un alcool aliphatique ayant au moins 4 atomes de carbone ou comme constituant d'un mélange de diluants, un alcool aliphatique ayant au moins deux atomes de carbone.

7. Procédé de production de semichlorhydrate de CCDC suivant la revendication 5, **caractérisé en ce que** lorsqu'on utilise un alcool aliphatique ayant au moins 3 atomes de carbone comme constituant d'un mélange de diluants, on utilise en même temps comme autre diluant la N-méthylpyrrolidone dans un rapport de 1:1 à 3:1.

8. Procédé de production de semichlorhydrate de CCDC suivant la revendication 6, **caractérisé en ce que** lorsqu'on utilise le propanol comme constituant d'un mélange de diluants, on utilise en même temps comme autre diluant le N,N-diméthylformamide dans un rapport de 1:1 à 3:1.

9. Médicament **caractérisé en ce qu'**il contient, à côté de substances auxiliaires et de supports classiques, du semichlorhydrate de CCDC suivant l'une des revendications 1 à 4.

10. Utilisation de semichlorhydrate de CCDC suivant l'une des revendications 1 à 4 pour la préparation de médicaments.

11. Semichlorhydrate de CCDC suivant l'une des revendications 1 à 4, destiné à être utilisé dans des agents antibactériens.
